(19) Europäisches Patentamt
European Patent Office
Office européen des brevets

(11) Veröffentlichungsnummer: **0 011 815**
**A1**

(12) # EUROPÄISCHE PATENTANMELDUNG

(21) Anmeldenummer: **79104619.6**

(22) Anmeldetag: **21.11.79**

(51) Int. Cl.$^3$: **C 07 C 65/03**
C 07 C 51/353

(30) Priorität: **02.12.78 DE 2852163**

(43) Veröffentlichungstag der Anmeldung:
**11.06.80 Patentblatt 80/12**

(84) Benannte Vertragsstaaten:
**BE CH DE FR GB IT NL**

(71) Anmelder: **BAYER AG**
**Zentralbereich Patente, Marken und Lizenzen**
**D-5090 Leverkusen 1, Bayerwerk(DE)**

(72) Erfinder: **Horstmann, Walter, Dr.**
**Eichenweg 17**
**D-5060 Bergisch-Gladbach 2(DE)**

(72) Erfinder: **Potthast, Ruthard, Dr.**
**Martin-Buber-Strasse 45**
**D-5090 Leverkusen 3(DE)**

(72) Erfinder: **Hammerström, Knut, Dr.**
**Pehlengarten 3**
**D-5060 Bergisch-Gladbach 1(DE)**

(54) **Verfahren zur Herstellung von m-Hydroxy-benzoesäure.**

(57) Die Erfindung betrifft ein Verfahren zur Herstellung von m-Hydroxy-benzoesäure bei dem man Carbonsäuren des Diphenyläthers im Temperaturbereich von 150 bis 400°C und bei Drucken von 0,1 bis 100 bar in Gegenwart von Hydroxyden umsetzt.

EP 0 011 815 A1

Croydon Printing Company Ltd.

0011815

BAYER AKTIENGESELLSCHAFT 5090 Leverkusen, Bayerwerk

Zentralbereich
Patente, Marken und Lizenzen Mn/Dä

## Verfahren zur Herstellung von m-Hydroxy-benzoesäure

Die Erfindung betrifft ein Verfahren zur Herstellung von m-Hydroxy-benzoesäure aus Mono- oder Dicarbonsäuren des Diphenyläthers.

Aus der US 3 366 691 ist bekannt, daß 4,4'-Diphenoxybenzophenon bei 300°C mit wäßrigem Alkali in 4,4'-Dihydroxy-benzophenon aufgespalten werden kann.

Es wurde ein Verfahren zur Herstellung von m-Hydroxy-benzoesäure gefunden, das dadurch gekennzeichnet ist, daß man Carbonsäuren des Diphenyläthers der Formel

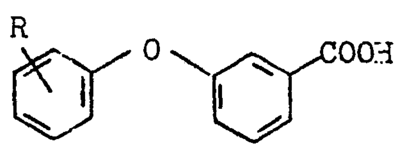

(I)

worin
R Wasserstoff, Methyl oder Carboxyl bedeutet,
oder deren Alkali- oder Erdalkalisalze,
im Temperaturbereich von 150 bis 400°C und bei Drucken von 0,1 bis 100 bar in Gegenwart von Hydroxyden umsetzt.

Das erfindungsgemäße Verfahren kann anhand der folgenden Reaktionsgleichung erläutert werden:

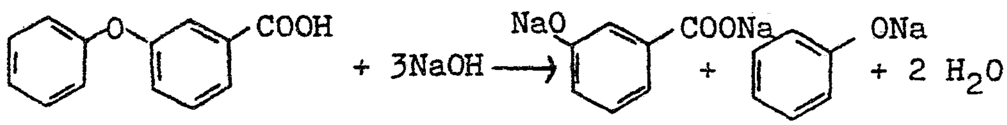


Nach dem erfindungsgemäßen Verfahren können 3-Phenoxy-benzoesäure und Dicarbonsäuren des Diphenyläthers, bzw. deren Alkali- oder Erdalkalisalze, die mindestens eine Carboxylgruppe in meta-Stellung zur Ätherbrücke enthalten, als Einzelverbindung oder im Gemisch in die m-Hydroxy-benzoesäure überführt werden. Ist der Rest R in Formel (I) Wasserstoff oder Methyl, so erhält man außerdem Phenol oder Kresole, die ebenfalls wirtschaftlich verwertet werden können.

Als Ausgangsprodukte für das erfindungsgemäße Verfahren seien beispielsweise genannt: 3-Phenoxy-benzoesäure, 4-Methyl-diphenyläther-3-carbonsäure, Diphenyläther-2',3-dicarbonsäure, Diphenyläther-3,3'-dicarbonsäure, Diphenyläther-3,4'-dicarbonsäure.

Nach dem erfindungsgemäßen Verfahren werden überraschenderweise bei der Hydrolyse isomerer Dicarbonsäuren des Diphenyläthers, wie etwa entstehende Salicylsäure oder 4-Hydroxy-benzoesäure, im Gegensatz zu m-Hydroxy-benzoesäure decarboxyliert, so daß nur Phenol und m-Hydroxy-benzoesäure entstehen, die leicht voneinander getrennt werden können, z.B. aufgrund ihrer unterschiedlichen Löslichkeiten in Wasser.

Die Mono- und Dicarbonsäuren des Diphenyläthers sind teilweise bekannt. So kann man z.B. 3-Phenoxy-benzoesäure in einfacher Weise durch Luftoxidation von 3-Phenoxitoluol in Gegenwart geeigneter Katalysatoren herstellen (DOS 2 604 474). Auf demselben Wege können die Dicarbonsäuren des Diphenyläthers durch Einsatz geeigneter Dimethyldiphenyläther hergestellt werden.

Das erfindungsgemäße Verfahren kann in kontinuierlicher oder diskontinuierlicher Arbeitsweise durchgeführt werden.

Das erfindungsgemäße Verfahren kann im allgemeinen im Temperaturbereich von 150 bis 450°C, vorzugsweise von 200 bis 300°C, durchgeführt werden. Das erfindungsgemäße Verfahren kann im allgemeinen im Autoklaven oder in einer Reaktionsrohrschlange bei Drucken bis zu 200 bar, insbesondere bei Drucken von 1 bis 80 bar, durchgeführt werden. Die Reaktionszeiten können in der Regel 1 bis 50 Stunden betragen.

Das erfindungsgemäße Verfahren wird in Gegenwart eines Hydroxydes durchgeführt. Als Hydroxyde seien beispielsweise die Alkali- und/oder Erdalkalihydroxyde, wie Natriumhydroxyd, Kaliumhydroxyd, Magnesiumhydroxyd und

Calciumhydroxyd genannt.

Das erfindungsgemäße Verfahren kann bevorzugt in Gegenwart von Wasser durchgeführt werden. Bezogen auf die eingesetzte Carbonsäure des Diphenyläthers kann der Anteil des Wassers das 1- bis 20-fache, bevorzugt das 2- bis 10-fache, betragen.

Bevorzugt für das erfindungsgemäße Verfahren sind Zusätze von Natrium- und/oder Kaliumacetat zu der Schmelze des Hydroxyds. Der Anteil des Natrium- und/oder Kaliumacetats zur Alkalischmelze kann 1 bis 100 Gew.-%, bevorzugt 5 bis 50 Gew.-%, bezogen auf das Alkali- oder Erdalkalihydroxid, betragen.

Das Molverhältnis des Diphenyläthers der Formel (I) zu dem Alkali- bzw. Erdalkalihydroxyd liegt im allgemeinen bei 1:3 bis 20 Mol, bevorzugt bei 1:5 bis 10 Mol.

Das erfindungsgemäße Verfahren kann beispielsweise wie folgt durchgeführt werden:
In einem Druckbehälter, beispielsweise einem Autoklaven, wird die zu spaltende Diphenyläthercarbonsäure in das vorgelegte, gegebenenfalls wäßrige, Hydroxyd eingetragen und unter Rühren auf die Reaktionstemperatur erhitzt; dabei stellt sich der Reaktionsdruck ein. Nach Beendigung der Umsetzung kühlt man das Reaktionsgemisch ab, und trennt evtl. nicht umgesetztes Ausgangsmaterial als Alkali- bzw. Erdalkalisalz ab und isoliert m-Hydroxybenzoesäure durch Ansäuern mit einer Mineralsäure aus

dem Filtrat. Gleichzeitig entstandenes Phenol oder Kresol gelangt in das saure Filtrat und kann gegebenenfalls durch Extraktion gewonnen werden.

Nach dem erfindungsgemäßen Verfahren wird m-Hydroxybenzoesäure in hoher Ausbeute und Reinheit erhalten.

Das erfindungsgemäße Verfahren besitzt u.a. gegenüber anderen Verfahren zur Herstellung von m-Hydroxy-benzoesäure, z.B. durch Alkalischmelze von m-Sulfobenzoesäure, den Vorteil, daß kein sulfithaltiges Abwasser entsteht.

m-Hydroxy-benzoesäure ist ein Zwischenprodukt zur Herstellung von Pflanzenschutzmitteln (US-P 4 031 131).

Beispiel 1

51,6 Teile Diphenyläther-3,3'-dicarbonsäure und 193 Teile 25 %ige Ätznatronlauge werden in einem 0,3 l fassenden Autoklaven aus Nickel 25 Stunden lang auf 270 bis 280°C erhitzt. Der Druck steigt dabei auf 37 bis 40 bar an. Nach dem Abkühlen filtriert man die erhaltene Reaktionsmischung und säuert das Filtrat mit ca. 130 g 30 %iger Chlorwasserstoffsäure bis zum pH-Wert von 1 an. Man isoliert den Niederschlag von m-Hydroxy-benzoesäure bei 20°C, wäscht mit 100 Teilen eiskaltem Wasser und trocknet im Vakuumschrank bei 60°C. Man erhält 48,5 Teile einer 99,3 %igen m-Hydroxy-benzoesäure vom Schmelzpunkt 201 bis 202°C.

Beispiel 2

15,5 Teile m-Phenoxy-benzoesäure und 174 Teile 10 %ige Natronlauge werden in einem 0,3 l Nickelautoklaven 15 Stunden lang auf 300°C erhitzt. Dabei stellt sich ein Druck von 68 bis 70 bar ein. Nach dem Abkühlen fällt man die m-Hydroxy-benzoesäure durch Zugabe von 49,5 Teilen 30 %iger Salzsäure aus, saugt bei 20°C ab und wäscht mit 30 Teilen Eiswasser nach. Nach dem Trocknen bei 60°C erhält man 9,5 Teile einer 97,1 %igen m-Hydroxy-benzoesäure. Aus den Filtraten kann man nach Extraktion mit Toluol und Verdampfen des Lösungsmittels noch 6 Teile Phenol gewinnen.

Beispiel 3

51,6 Teile eines Gemisches von 20 Gew.-% 2,3'-, 35 Gew.-% 3,3'-, 31 Gew.-% 3,4'- und 14 Gew.-% 4,4'-Diphenyläther-dicarbonsäure werden, wie im Beispiel 1 beschrieben, mit Ätznatronlauge hydrolysiert. Nach entsprechender Aufarbeitung erhält man 26 Teile m-Hydroxybenzoesäure. Die isomeren Hydroxy-benzoesäuren werden zu Phenol decarboxiliert, das man aus den Filtraten gewinnen kann.

Beispiel 4

41,1 Teile Diphenyläther-3,3'-dicarbonsäure und 178,5 Teile 50 %ige Natronlauge werden in einem 0,3 l Nickelautoklaven 15 Stunden lang auf 275°C erhitzt. Dabei stellt sich ein Druck von 18 bar ein. Nach dem Abkühlen filtriert man die erhaltene Reaktionsmischung und säuert das Filtrat mit 236 Teilen 30 %iger Salzsäure bis zum pH-Wert von 0,5 an. Man isoliert den Niederschlag von m-Hydroxy-benzoesäure bei 20°C, wäscht mit 100 Teilen eiskaltem Wasser und trocknet im Vakuumschrank bei 60°C. Man erhält 40,5 Teile einer 99,75 %igen m-Hydroxy-benzoesäure.

Beispiel 5

18,7 Teile Diphenyläther-2,3'-dicarbonsäure und 174 Teile 10 %ige Natronlauge werden in einem 0,3 l fassenden Autoklaven aus Nickel 10 Stunden lang auf 300°C erhitzt. Der Druck steigt dabei auf 70 bis 72 bar an. Nach dem Abkühlen fällt man die m-Hydroxy-benzoesäure durch Zugabe von 57 Teilen 30 %iger Salzsäure aus, saugt bei 20°C ab, und wäscht mit 25 Teilen eiskaltem Wasser nach. Nach dem Trocknen bei 60°C erhält man 8,7 Teile einer 96,1 %igen m-Hydroxy-benzoesäure.
Aus den Filtraten kann man nach Extraktion mit Toluol das durch Decarboxylierung entstandene Phenol gewinnen.

Beispiel 6

Eine Lösung von 258 Teilen (1 Mol) Diphenylätherdicarbonsäure-3,3' in 4800 Teilen 5 %iger Natronlauge wird kontinuierlich unter Druckkonstanthaltung (ca. 200 bar Reaktionsdruck) durch ein auf 350°C erwärmtes Nickelrohr gepumpt. Die Pumpgeschwindigkeit ist so bemessen, daß eine Verweilzeit von etwa 2 Stunden resultiert. Die Reaktionslösung wird nach dem Entspannen auf Raumtemperatur gekühlt, filtriert und unter weiterem Kühlen mit konzentrierter Salzsäure auf pH 1 eingestellt. Die Fällung wird anschließend abgesaugt, mit etwa 500 ml kaltem Wasser nachgewaschen und getrocknet.
Ausbeute: 235 g m-Hydroxy-benzoesäure (85,1 % der Theorie)
Schmelzpunkt: 200 bis 201°C

Patentansprüche:

1. Verfahren zur Herstellung von m-Hydroxy-benzoesäure, dadurch gekennzeichnet, daß man Carbonsäuren des Diphenyläthers der Formel

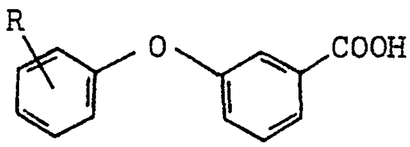

worin
R Wasserstoff, Methyl oder Carboxyl bedeutet,
oder deren Alkali- oder Erdalkalisalze
im Temperaturbereich von 150 bis 400°C und bei Drucken von 0,1 bis 100 bar in Gegenwart von Hydroxyden umsetzt.

2. Verfahren nach Anspruch 1, dadurch gekennzeichnet, daß es in Gegenwart von Wasser durchgeführt wird.

3. Verfahren nach den Ansprüchen 1 und 2, dadurch gekennzeichnet, daß es in Gegenwart von Natrium- und/oder Kaliumacetat durchgeführt wird.

4. Verfahren nach den Ansprüchen 1 bis 3, dadurch gekennzeichnet, daß man Alkalihydroxyde oder Gemische von Alkalihydroxyden einsetzt.

5. Verfahren nach den Ansprüchen 1 bis 4, dadurch gekennzeichnet, daß man die Diphenyläthercarbonsäure und das Alkalihydroxyd im Molverhältnis 1:3 bis 20 einsetzt.

6. Verfahren nach den Ansprüchen 1 bis 5, dadurch gekennzeichnet, daß man Isomerengemische von Diphenylätherdicarbonsäuren einsetzt, bei denen zumindest eine Carboxylgruppe in 3-Stellung steht.

7. Verfahren nach den Ansprüchen 1 bis 6, dadurch gekennzeichnet, daß man als Carbonsäure des Diphenyläthers die 3,3'-Diphenyläther-dicarbonsäure einsetzt.

0011815

Nummer der Anmeldung

Europäisches Patentamt

# EUROPÄISCHER RECHERCHENBERICHT

EP 79 10 4619

| Kategorie | EINSCHLÄGIGE DOKUMENTE — Kennzeichnung des Dokuments mit Angabe, soweit erforderlich, der maßgeblichen Teile | betrifft Anspruch |
|---|---|---|
| A | US - A - 2 439 375 (SCHWENK et al) * Anspruch 1; Spalte 5, Beispiel V * -- | 1 |
| A | DE - A - 876 844 (BASF) * Anspruch; Beispiel 3 * ---- | 1 |

KLASSIFIKATION DER ANMELDUNG (Int.Cl. 3)

C 07 C 65/03
51/353

RECHERCHIERTE SACHGEBIETE (Int. Cl. 3)

C 07 C 65/00
65/01
65/03
51/353
51/41

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 18-02-1980 | KLAG |

EPA form 1503.1 06.78